Europäisches Patentamt

European Patent Office

Office européen des brevets

(11) Publication number: **0 291 950 B1**

(12) **EUROPEAN PATENT SPECIFICATION**

(45) Date of publication of patent specification: **12.08.92**  (51) Int. Cl.⁵: **C07C 25/28**, C07C 17/42

(21) Application number: **88107955.2**

(22) Date of filing: **18.05.88**

(54) Process and composition for stabilization of ar-brominated styrenic monomer against premature polymerization.

(30) Priority: **20.05.87 US 52525**

(43) Date of publication of application:
**23.11.88 Bulletin 88/47**

(45) Publication of the grant of the patent:
**12.08.92 Bulletin 92/33**

(84) Designated Contracting States:
**DE ES FR GB IT NL**

(56) References cited:
EP-A- 0 159 915
US-A- 2 965 685
US-A- 4 376 221

PATENT ABSTRACTS OF JAPAN, unexamined applications, C field, vol. 11, no. 13, January 14, 1987; THE PATENT OFFICE JAPANESE GOVERNMENT, page 69C 397

(73) Proprietor: **GE CHEMICALS, INC.**
**Parkersburg Center 5th & Avery Streets**
**Parkersburg, West Virginia 26102(US)**

(72) Inventor: **Campbell, Stephen Michael**
**Rt- 1, Box 43, New England**
**West Virginia 26181(US)**
Inventor: **Wozny, John Carl**
**P.O. Box 112**
**Coolville Ohio 45723(US)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to prevention of premature polymerization of ar-brominated styrene monomer.

Vinyl aromatic comopunds tend to polymerize on standing. During the manufacture, shipping, and storage of these compounds, inhibitors are normally added to prevent or highly retard polymerization until such time that these compounds are intentionally converted to polymers.

Dozens of stabilizing agents for prevention of undesirable polymerization of monomers in general have been reported in the literature. As noted by Czechoslovakian Patent Document No. 163,428 (Konecny et al) compositions which are known to stablize one or more monomers include: sulfur, copper, silver, gold, activated carbon, triphenylarsine, $NH_3$, diazoaminobenzene, some diolefins, phenylacetylene, sym-trinitrobenzene, p-benzoquinone, acetaldehyde, aniline condensates, N,N′-dibutyl-o-phenylenediamine,N-butyl-p-aminophenol, 2,4,6-triphenylphenoxyl, pyrogallol, pyrocatechol, hydroquinone, alkyl-substituted pyrocatechols, dialkylhydroquinone, 2,4,6-di-chloronitrophenol, halogen-ortho-nitrophenols, alkoxyhydroquinone, mono-, di-, and polysulfides of phenols and pyrocatechols, aromatic nitrocompounds, amines, thiols, oximes or hydrazones of quinone, phenothiazine, dialkylhydroxylamines, and nitro compounds. However, as also noted by Konecny et al only a few of the known stabilizers are commercially employed with benzoquinone, hydroquinone, and tert-butylpyrocatechol mentioned by name. The other stabilizers have various shortcomings including undesirable levels of toxicity, explosiveness, or insufficient stabilizing activity. Also, many of the above listed compounds are noted to be stabilizers in conjunction with only a limited group of monomers. Konecny et al suggest use of a synergistic mixture of 2,4 dinitro-o-cresol and diethylhydroxylamine with styrene and divinyl benzene.

In 1960 a process for stabilizing vinyl aromatics such as styrene or ring chlorinated styrene was described in U. S. Patent 2,965,685 (Campbell) which utilized N,N dialkylhydroxylamines such as N,N-diethylhydroxylamine (DEHA). Recommended levels of inhibitor are said to be from about 0.001% to about 5% by weight.

In 1966 aliphatic carboxylic acid salts of DEHA were described as stabilizers for aromatic vinyl compounds such as styrene in U. S. Patent 3,248,440 (Albert).

While diethylhydroxylamine-containing compositions have been described as among a plethora of types of chemicals useful for stabilizing vilnyl aromatic compounds such as styrene, its use with brominated styrenes has not been addressed. Only a few compositions have been described as useful for stabilizing ar-brominated styrenes such as dibromostyrene.

In British Patent Document 1,230,979, dibromostyrene (DBS) is described as being stabilized by picric acid or by a mixture of picric acid with a quinone or phenol such as hydroquinone, benzoquinone and t-butyl catechol. Unfortunately picric acid has the undesirable characteristics of coloring monomers bright yellow, and when concentrated, being shock sensitive.

Also, U. S. Patent 4,276,189 (Jackisch) describes a process for retarding polymerization of dibromostyrene by addition of a metal oxide such as magnesium, calcium or zinc oxide with or without an additional stabilizing agent such as 4-tert-butylcatechol and most benzoquinones.

According to the present invention, a novel process for stabilizing ar-brominated styrene monomer against polymerization is described in which ar-brominated styrene monomer such as mono-, di-, or tri-bromostyrene or mixtures thereof are contacted or admixed with a stabilizing or polymerization retarding amount of N,N dialkylhydroxylamine to produce stabilized monomer.

Surprisingly, amounts of N,N diethylhydroxylamine (DEHA) as low as 500 ppm will stabilize ar-brominated styrene monomer for a period of 300 hours at temperatures at or below 50°C.

The inventive process produces a stabilized ar-brominated styrene monomer composition which resists premature polymerization which may be stored, handled, transported, and subsequently polymerized with a greater deal of control, safety and economy relative to unstabilized monomer. In particular, dibromostyrene monomer may be maintained for a longer period of time without undesirable polymerization.

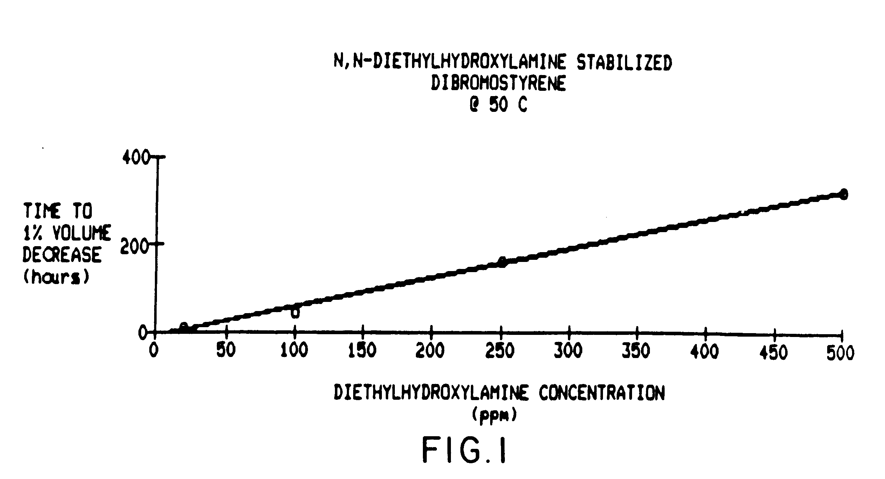

Fig. 1 is a graph comparing DEHA concentration against time required to show a one percent volume decrease.

The process of the present invention is useful to stabilize ar-brominated styrene monomer against premature polymerization. It is desirable to prevent or retard the rate of polymerization in order to ship or store monomer until required for use. Brominated styrenes such as dibromostyrene have been suggested as a flame retardant monomer for use in making flame retardant materials.

There are several reasons why, in spite of the large number of inhibitors that have been discovered for vinyl aromatic compounds including diethylhydroxylamine, so few would be expected to find applicability with dibromostyrene.

Brominated styrenes have shown a markedly greater tendency to polymerize than styrene monomer alone. Table 1 shows polymerization constants ($K_p$) and termination constants ($K_t$) for styrene and monosubstituted halostyrene monomers in dimethylacetamide at about 30 degrees C as published by Imoto et al, Makromol, Chem., (1965), 86, 217.

## TABLE 1

| Monomer | $K_p$ 1/mole sec | $K_t$ X $10^6$ 1/mole sec |
|---|---|---|
| Styrene | 106 | 108 |
| Styrene p-F | 112 | 127 |
| Styrene p-Cl | 150 | 77 |
| Styrene p-Br | 186 | 46 |

As shown in Table I, the rates of polymerization ($K_p$) for styrene and substituted styrene monomers indicates that halogenated styrenes are significantly more reactive than unsubstituted styrene and therefore more difficult to stabilize against premature polymerization. Also, since the termination rate ($K_t$) for an active bromostyrene radical is lower than that for styrene or its p-F or p-Cl analogs, the brominated styrenes are especially difficult to stabilize. With a lower termination rate ($K_t$) a greater amount of polymer is formed for each active free radical than for monomers having high $K_t$ values. Cubbon and Smith in their article entitled, "The Properties of Nuclear Brominated Styrenes I - The Synthesis and Polymerization of Dibromostyrene and Tribromostyrene," Polymer, Vol. 10, no. 7 (1969) pp 479-487, have shown in their Fig. 1 the polymerization rate of dibromostyrene to be more than 10 times as active as styrene.

It is also noted that dibromostyrene formed by conventional processes may be a mixture of mono-, di- and tribromostyrenes. Tribromostyrene has an even greater tendency to polymerize than the highly reactive dibromostyrenes. Cubbon and Smith, supra, further state the order of rates of thermal polymerization to be: 2,4,5-tribromostyrene > 2,4-and 3,4 dibromostyrene ≫ styrene.

Many known stabilizers for styrene are ineffective or poor polymerization inhibitors for substituted styrenes. Also, many free radical stabilizers are effective at high temperatures, but do not follow a first order stability relationship to room temperature. Therefore, the stabilizing capacity of inhibitors should be determined at or near the actual conditions contemplated.

Following are examples given to illustrate the process and compositions of the invention. Evidence of polymerization was followed by the dilitometric method. All examples were conducted at 50°C and at atmospheric pressure. The experimental methodology was as follows:

Monomer samples were prepared for testing by introducing a precisely known weight of a test inhibitor into a clean 9 inch Kimble 2075C cream test bottle. (A cream test bottle consists of a large flat bottomed bulb which tapers into a long narrow neck. The neck is calibrated in arbitrary units from 0 to 50.) A known weight of monomer was then added in portions with intermittent mixing to yield the indicated concentration of the test substance in the monomer. Each bottle was stoppered and mixing was completed by inverting the bottle 25 times. (The volumes were precalculated to yield a level of about 25 on the scale.) The stopper was removed and the bottle was loosely covered with aluminum foil to eliminate evaporation. The above procedure and use of a long narrow necked bottle acts to minimize air exposure. Minimum air exposure is desirable as an experimental condition because it simulates reasonable expected static storage conditions for bulk handling of this monomer. The bottles were immersed into a constant temperature bath at 50.00° +/-0.02°C and allowed to equilibrate. After equilibration, the volume was readjusted to exactly 25.0 on the bottle scale. The volume change was recorded versus time to monitor any conversion to polymer. A 5 unit change on the bottle scale was determined to be equal to a 1% change in total volume at midscale.

The styrene used in the exmaples was commercially available and used as received from Cosden Chemical Company. The dibromostyrene was obtained as an experimental monomer mixture of monobromostyrene, dibromostyrene, and tribromostyrene from Great Lakes Chemicals under the name of "Dibromostyrene" and from Ethyl Corporation under the name of Satex RB-25. No difference between the two monomers was observed with respect to stabilizer response. N,N-diethylhydroxylamine was purchased from Aldrich Chemical Company at 97% purity under the product designation of D 9720-7.

The results are shown in Table 2 for ease of comparison. Examples 1-3 and 8 are comparative examples and examples 4-7 and 9 are of the present invention.

## TABLE 2

| Example | Inhibitor | Hours to % Volume Decrease @ 50°C | | |
|---------|-----------|------|------|------|
| | | 0.2% | 0.5% | 1.0% |
| 1. Commercial STYRENE | 10 ppm t-BC | 50 | 131 | 310 |
| 2. DIBROMOSTYRENE | No stabilizer | 1.5 | 3 | 7 |
| 3. DIBROMOSTYRENE | 250 ppm t-BC | 10 | 21 | 27 |
| 4. DIBROMOSTYRENE | 20 ppm DEHA | | | 15 |
| 5. DIBROMOSTYRENE | 100 ppm DEHA | 19 | 28 | 52 |
| 6. DIBROMOSTYRENE | 250 ppm DEHA | 40 | 99 | 180 |
| 7. DIBROMOSTYRENE | 500 ppm DEHA | 60 | 145 | 320 |
| 8. DIBROMOSTYRENE* | 250 ppm t-BC | | | 0.4 |
| 9. DIBROMOSTYRENE* | 500 ppm DEHA + 250 ppm t-BC | | | 1.5 |

**\* Polymerization intentionally begun with initiator**

### Example 2 (Control - Not of the Invention)

As shown by the data in example 2 of Table 2, dibromostyrene monomer, without any stabilizing agent, quickly polymerizes until a one percent drop in volume occurs after only seven hours. Thus, without inhibitor the original monomer quickly converts to polymer necessitating costly removal steps and rendering that portion of the monomer unsuitable for its intended purpose. The stability against premature polymerization of unstabilized dibromostyrene (DBS) may vary from one to several hours at 50°C when tested with minimum exposure to oxygen from the air.

### Examples 1 and 3 (Not of the Invention)

Examples 1, and 3 in Table 2 show that dibromostyrene stabilized with 250 ppm t-butyl catechol (t-BC) is less than one tenth as stable as the commercial styrene sample containing only 10 ppm t-butyl catechol as measured by a 1% volume decrease. While t-BC added to dibromostyrene will provide some degree of stabilization, it is not comparable to the level of stabilization of styrene with t-BC.

### Examples 4-7

Examples 4, 5, 6 and 7 in Table 2 show that the induction periods before undesirable polymerization (1% volume decrease) for 500 ppm, 250 ppm, 100 ppm, and 20 ppm N,N-diethylhydroxylamine in dibromostyrene were 320, 180, 52 and 15 hours respectively. Fig. 1 demonstrates that the relationship between stability and concentration was essentially linear. A stabilizing amount is defined as sufficient added inhibitor to stop or retard polymerization for a time exceeding that observed for a monomer without added inhibitor.

4

Comparison of Examples 3 and 6 in Table 2 shows that DEHA is a superior stabilizer for dibromystyrene relative to t-butylcatechol. At equal concentrations by weight the DEHA was four to six times as effective as t-butylcatechol. When calculated on a molar basis the DEHA is as much as 12 times as efficient at minimizing polymer formation as t-butylcatechol.

Examples 8-9

Examples 8 and 9 demonstrate the ability to initiate polymerization when desired by addition of a polymerization initiator. DBS containing 500 ppm N,N-diethylhydroxylamine and 250 ppm t-BC began rapidly polymerizing in 1.5 hours when initiated in bulk with a low radical flux polymerization initiator such as 0.1% by weight of a commercially available initiator sold under the trade name VAZO 52 by E. I. Dupont de Nemours Co. at 50 C. DBS containing only the t-BC exhibited rapid polymerization onset after 0.4 hours under the same conditions. In both cases polymerization continued to form a glassy solid homopolymer.

The high reactivity of dibromostyrene toward polymerization tends to limit the storage stability and handling latitude of this monomer. Surprisingly, stabilization of the dibromostyrene with a dialkylhydroxylamine such as diethylhydroxylamine shows that dibromostyrene may be stabilized to a level comparable to commercial styrene. Also, N,N-diethylhydroxylamine inhibitor has the added advantage that it can be readily overcome by free radical initiators when used at levels which provide adequate storage stability in dibromostyrene. An upper allowable N,N-diethylhydroxylamine concentration appears to be limited only by any retarding effect on polymerizability during reaction conditions. Sufficient storage stability is achieved in DBS at N,N-diethylhydroxylamine concentrations well below the level where polymerizability is adversely affected.

Other dialkylhydroxylamines are believed to be suitable, especially linear, branched-chain or cyclic diloweralkylhydroxylamines having one to seven carbon atoms.

## Claims

1. A process for stabilizing ar-brominated styrene monomer against polymerization, comprising admixing 2 ppm to 50,000 ppm of N,N-dialkylhydroxylamine with the ar-brominated styrene monomer to maintain the monomer at a level of 99 % of its original volume for a period of 300 hours at a temperature at or below 50°C.

2. A process as defined in claim 1 wherein said N,N-dialkylhydroxylamine is N,N-diethylhydroxylamine.

3. A process as defined in any one of claims 1 and 2 wherein said stabilizing amount is from 2 ppm to 10,000 ppm.

4. A process as defined in any one of claims 1 and 2 wherein said stabilizing amount is from 20 ppm to 5,000 ppm.

5. A process as defined in any one of claims 1 to 4 wherein said ar-brominated styrene monomer comprises dibromostyrene.

6. A process as defined in any one of claims 1 to 5 wherein said ar-brominated styrene monomer comprises a mixture of ar-brominated styrenes forming a composition having from 0.8 to 3.2 bromines per aromatic ring.

7. A stabilized ar-brominated styrene monomer composition comprising an ar-brominated styrene monomer admixed with from 2 ppm to 50,000 ppm of N,Ndialkylhydroxylamine.

8. A stabilized ar-brominated styrene monomer composition as defined in claim 7 wherein said N,N-dialkylhydroxylamine is N,N-diethylhydroxylamine.

9. A composition as defined in claim 7 wherein said N,N-dialkylhydroxylamine is present in an amount between 2 ppm to 10,000 ppm by weight.

10. A composition as defined in any one of claims 7 to 9 wherein said ar-brominated styrene monomer comprises dibromostyrene.

**11.** A composition as defined in any one of claims 7 to 10 wherein said ar-brominated styrene monomer comprises a mixture of mono-, di-, and tri- bromostyrene.

**Revendications**

**1.** Procédé de stabilisation de monomère de styrène bromé sur le noyau aromatique contre la polymérisation, comprenant le mélange de 2ppm à 50 000ppm de N,N-di-alkylhydroxylamine avec le monomère de styrène bromé sur le noyau aromatique pour maintenir le monomère à un niveau de 99% de son volume originel pendant une période de 300 heures à une température à ou inférieure à 50°C.

**2.** Procédé selon la revendication 1 dans lequel la N,N-dialkylhydroxylamine est la N,N-diéthylhydroxylamine.

**3.** Procédé selon la revendication 1 ou 2 dans lequel la quantité stabilisante est de 2ppm à 10 000ppm.

**4.** Procédé selon la revendication 1 ou 2 dans lequel la quantité stabilisante est de 20ppm à 5 000ppm.

**5.** Procédé selon l'une quelconque des revendications 1 à 4 dans lequel le monomère de styrène bromé sur le noyau aromatique contient dibromostyrène.

**6.** Procédé selon l'une quelconque des revendications 1 à 5, dans lequel le monomère de styrène bromé sur le noyau aromatique comprend un mélange de styrènes bromés sur le noyau aromatique formant une composition ayant de 0,8 à 3,2 atomes de brome par noyau aromatique.

**7.** Composition de monomère de styrène bromé sur le noyau aromatique stabilisé comprenant un monomère de styrène bromé sur le noyau aromatique mélangé à 2ppm à 50000 ppm de N,N-diaklylhydroxylamine.

**8.** Composition de monomère de styrène bromé sur le noyau aromatique stabilisé selon la revendication 7 dans laquelle la N,N-dialkylhydroxylamine est la N,N-diéthylhydroxylamine.

**9.** Composition de monomère de styrène bromé sur le noyau aromatique stabilisé selon la revendication 7 dans laquelle la N,N-dialkylhydroxylamine est en une quantité comprise entre 2ppm et 10 000ppm en poids.

**10.** Composition selon l'une quelconque des revendications 7 à 9 dans laquelle le monomère de styrène bromé sur le noyau aromatique contient dibromostyrène.

**11.** Composition selon l'une quelconque des revendications 7 à 10 dans laquelle le monomère de styrène bromé sur le noyau aromatique contient un mélange de mono-, di-, et tri-bromostyrène.

**Patentansprüche**

**1.** Verfahren zum Stabilisieren von ar-bromierten Styrolmonomeren gegen Polymerisation, **dadurch gekennzeichnet,** daß man 2 ppm bis 50 000 ppm eines N,N-Dialkylhydroxylamins zu dem ar-bromierten Styrolmonomer beimischt, um das Monomer für eine Zeitdauer von 300 Stunden bei einer Temperatur von 50°C oder weniger auf 99 % seines ursprünglichen Volumens zu halten.

**2.** Verfahren nach Anspruch 1, **dadurch gekennzeichnet,** daß das N,N-Dialkylhydroxylamin N,N-Diethylhydroxylamin ist.

**3.** Verfahren nach einem der Ansprüche 1 und 2, **dadurch gekennzeichnet,** daß die stabilisierende Menge in einem Bereich von 2 ppm bis 10 000 ppm liegt.

**4.** Verfahren nach einem der Ansprüche 1 und 2, **dadurch gekennzeichnet,** daß die stabilisierende Menge in einem Bereich von 20 ppm bis 5000 ppm liegt.

**5.** Verfahren nach einem der Ansprüche 1 bis 4, **dadurch gekennzeichnet,** daß das ar-bromierte

6

Styrolmonomer Dibromstyrol enthält.

6. Verfahren nach einem der Ansprüche 1 bis 5, **dadurch gekennzeichnet,** daß das ar-bromierte Styrolmonomer eine Mischung von ar-bromierten Styrolen umfaßt, so daß sich eine Zusammensetzung von 0,8 bis 3,2 Bromatomen pro aromatischem Ring ergibt.

7. Stabilisierte Zusammensetzung von ar-bromierten Styrolmonomeren, **gekennzeichnet durch** einen Gehalt von 2 ppm bis 50 000 ppm eines N,N-Dialkylhydroxylamins.

8. Stabilisierte Zusammensetzung von ar-bromierten Styrolmonomeren nach Anspruch 7, **dadurch gekennzeichnet,** daß das N,N-Dialkylhydroxylamin N,N-Diethylhydroxylamin ist.

9. Zusammensetzung nach Anspruch 7, **dadurch gekennzeichnet,** daß das N,N-Dialkylhydroxylamin in einer Menge von 2 ppm bis 10 000 ppm, bezogen auf das Gewicht, vorhanden ist.

10. Zusammensetzung nach einem der Ansprüche 7 bis 9, **dadurch gekennzeichnet,** daß das ar-bromierte Styrolmonomer Dibromstyrol umfaßt.

11. Zusammensetzung nach einem der Ansprüche 7 bis 10, **dadurch gekennzeichnet,** daß das ar-bromierte Styrolmonomer eine Mischung von Mono-, Di-, und Tri-bromstyrolen umfaßt.

N,N-DIETHYLHYDROXYLAMINE STABILIZED
DIBROMOSTYRENE
@ 50 C

TIME TO
1% VOLUME
DECREASE
(hours)

DIETHYLHYDROXYLAMINE CONCENTRATION
(ppm)

FIG.I

EP 0 291 950 B1